Europäisches Patentamt

**European Patent Office**

Office européen des brevets

(11) Publication number: **0 230 040 B1**

# EUROPEAN PATENT SPECIFICATION

(45) Date of publication of patent specification: **24.04.91** (51) Int. Cl.⁵: **A61M 25/00**

(21) Application number: **86117914.1**

(22) Date of filing: **22.12.86**

(54) **Catheter.**

(30) Priority: **16.01.86 SE 8600188**

(43) Date of publication of application:
**29.07.87 Bulletin 87/31**

(45) Publication of the grant of the patent:
**24.04.91 Bulletin 91/17**

(84) Designated Contracting States:
**BE CH DE ES FR GB IT LI NL**

(56) References cited:
**EP-A- 0 092 927**
**DE-B- 2 233 293**
**US-A- 4 129 129**
**US-A- 4 300 550**

(73) Proprietor: **GAMBRO AB**
**Post Box 10101**
**S-220 10 Lund(SE)**

(72) Inventor: **Claren, Jan Seivert**
**Protokollgränden 38**
**S-222 47 Lund(SE)**

(74) Representative: **Boberg, Nils Gunnar Erik**
**Gambro AB Patent Department Box 10101**
**S-220 10 Lund(SE)**

Rank Xerox (UK) Business Services

## Description

## TECHNICAL FIELD

The present invention relates to a catheter, comprising at least one inlet opening at its one end, at least one outlet opening at its other end and at least one intermediate opening in between which is adapted so that it can be closed whilst the said inlet and outlet openings are kept open, the said intermediate opening or intermediate openings being arranged on an essentially cylindrical part of the catheter.

The catheter is intended in particular to be used as a cardiac catheter for the withdrawal of blood from the "inferior vena cava" on the one hand and from the right auricle of the heart and/or the "superior vena cava" on the other hand.

## BACKGROUND ART

A catheter intended for the above mentioned use is described, for example, in the American patent specification 4 129 129. However, the use of the catheter described is relatively troublesome, among other things, owing to a part of the same having to be removed in order to achieve the desired flow through the different openings. Moreover, the design is unhandy, since it is relatively large and at the same time essentially stiff.

Please compare also DE-AS-22 33 293 describing a blood pump having a construction similar to the above-identified catheter but being intended for a quite different use.

## DISCLOSURE OF INVENTION

It is the object of the present invention to provide a catheter of the type defined above in general with essentially the same function as the known catheter, but without its disadvantages. This is achieved in that a slide is arranged preferably axially displaceable thereover for the opening and closing of this intermediate opening or these openings. Through such a design the slide becomes readily accessible and does not have to be removed from the catheter in connection with the keeping open of the intermediate openings.

The slide preferably is in the form of a cylindrical sleeve of the same cross-sectional shape as the said cylindrical part, which in principle can have any cross-sectional shape, but preferably is of circular-cylindrical shape. As a result a simple design is obtained, at the same time as it will be easy to ensure that the slide can not detach itself from the remainder of the catheter.

To facilitate the displacement of the slide it may be provided with a gripping part, e g a radially projecting part of flange.

To facilitate the handling of the slide the actual catheter may be provided with at least one stop element determining one end position of the slide.

In a preferred embodiment of the catheter its inlet end is designed as a circular-cylindrical part of a smaller diameter, formed preferably by heating and compressing or shrinking of the one end of an otherwise uniformly thick blank, whose other end is intended to form the remainder of the actual catheter.

The above mentioned stop element is produced preferably by means of upsetting of a heated part of the actual catheter to the shape of an all-around, radially outwards directed flange.

In order to facilitate the handling of the catheter as a whole the said other end is shaped so that it is directed or is directable at an angle in relation to its said first end. It may, for example, be made bendable with the help of a bellows-like intermediate part.

The catheter normally is connected to a flexible tube system filled with priming fluid. For this reason it is appropriately provided with an evacuating arrangement for the withdrawal of any air possibly accumulated between the blood and the priming fluid introduced. If in such a case the evacuating arrangement is constituted of a check valve, the bellows-like intermediate part may be used as a pump for pressing out the accumulated air.

Alternatively the evacuating arrangement may be constituted of an injeciton port or the like which is adapted so that it can be coupled to an external pump. It may comprise, for example, an elastic membrane, plug or the like which is adapted so that it can be pierced through by an injection syringe or the like used as a pump.

The catheter in accordance with the invention is used in particular as a cardiac catheter for the withdrawal of blood from the "inferior vena cava" and from the right auricle of the heart and/or the "superior vena cava". The slide is used in this case for the closing of the intermediate opening or intermediate openings as the catheter is introduced into, and/or taken out of, the heart respectively.

## BRIEF DESCRIPTION OF THE DRAWINGS

Two preferred embodiments of the subject to the invention are described in detail in the following with reference to the attached drawings.

Fig 1 shows a first embodiment in a position of use.

Fig 2 shows a side view of the same on a slightly enlarged scale.

Fig 3 shows a similar side view, partly in section.

Fig 4 and 5 show a section along the line IV-IV and the actual catheter tip seen in direction of the arrows V-V in fig 2 respectively.

Fig 6 shows a second embodiment of the subject of the invention in a position of use.

Fig 7-10, finally, show views corresponding to fig 2-5.

EMBODIMENT I

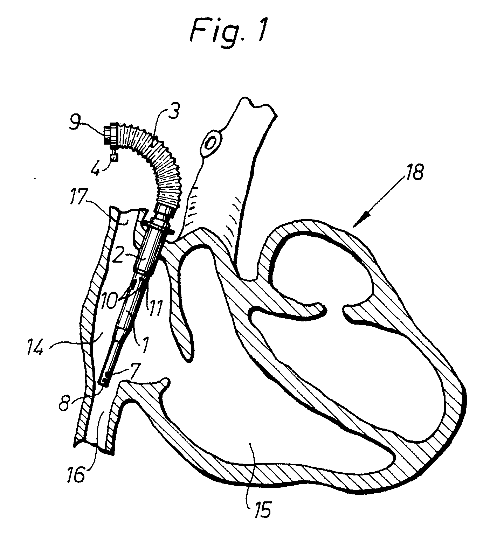

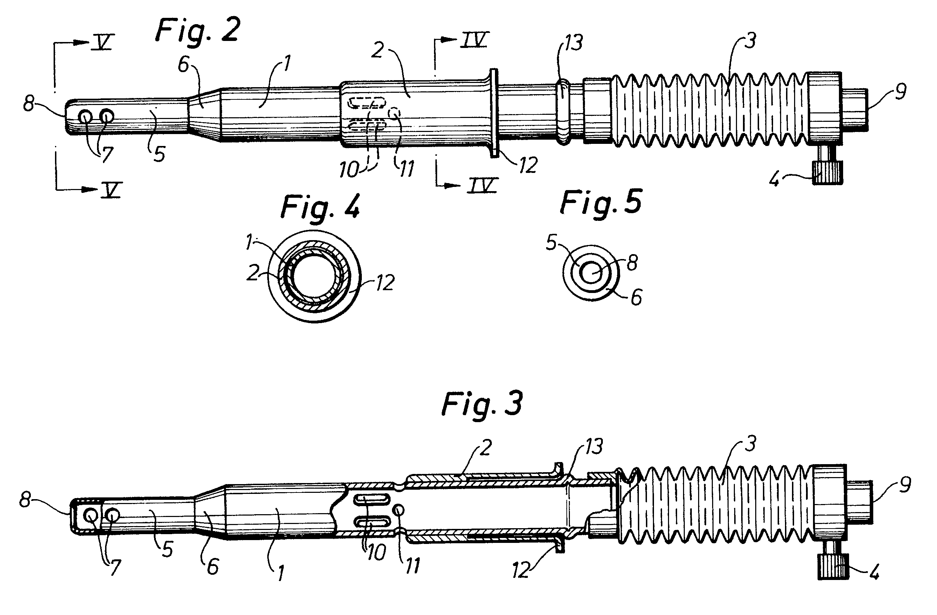

The embodiment shown in fig 1-5 comprises a main part 1 with a slide 2 displaceable thereon and a bellows-like part 3 connected thereto which is combined with an evacuating arrangement 4. The front part 5 of the main part 1 is designed with a smaller diameter and is connected to the remainder of the main part via a conical intermediate section 6. In the front part 5 a number of inlet openings 7 and 8 respectively are provided. At the opposite end of the catheter an outlet opening 9 is arranged instead which is intended to be connected, for example, to a flexible tube system (not shown) for connection to an oxygenator or the like.

Between the inlet openings 7 and 8 and the outlet opening 9 intermediate openings 10 and 11 respectively are provided on the main part 1, the firstnamed of which are oblong whereas the lastnamed are circular. These openings are covered by the displaceable slide whose displacement is facilitated by means of a gripping flange 12. A fully open position in this case is defined in that the gripping flange is guided towards a stop flange 13 formed by upsetting of the actual main part 1. At the same time this gripping flange marks the maximum depth of insertion of the catheter into the heart.

The bellows-like part 3 can be attached to the main part 1 with the help, for example, of glueing or welding. Alternatively these two parts may be made in one piece with the help of a technique known in itself.

How the catheter in accordance with the invention is intended to be applied in practice is described in detail in the above mentioned American patent 4 129 129. The application is illustrated, however, in fig 1 where 14 designates the right auricle, 15 the right ventricle, 16 the "inferior vena cava" and the right auricle. The catheter is appropriately brought into working position in straight, substantially vertical position, with the openings 10 and 11 covered by the slide 2. In this manner blood entering through the inlet openings 7 and 8 is prevented from flowing through the intermediate openings 10 and 11 before these have pushed through the intermediate wall. During this the blood will push up the catheter to a certain level, but normally not up to the outlet opening 9. Subsequently the remaining space can be filled, wholly or partly, with priming fluid, that is to say a fluid miscible with the blood, before the catheter is connected to a flexible tube system filled with similar priming fluid, which may be connected, for example, to an oxygenator. Any air entrapped in the process can be removed with the help of the evacuating arrangement 4. This may be constituted, for example, of a check valve through which the air is pumped out with the help of the bellows-like portion 3 used as a pump. Alternatively the evacuating arrangement 4 may be constituted of a so-called injection port which may comprise an elastic membrane, a plug or the like which is adapted so that it can be pierced through by an injection syringe or the like with the help of which the entrapped air can be extracted. Numeral 18 in fig 1, finally, designates the heart as a whole.

EMBODIMENT II

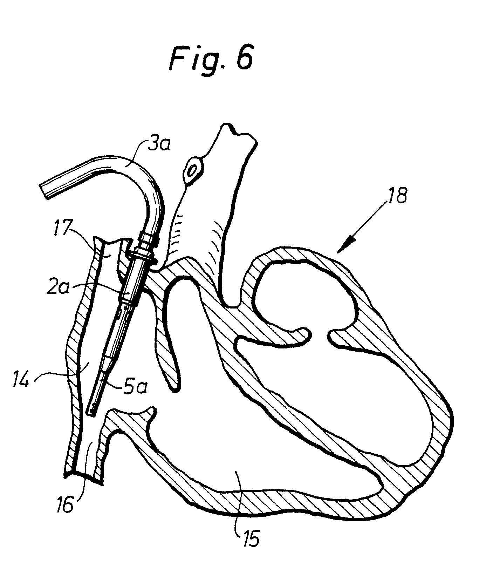

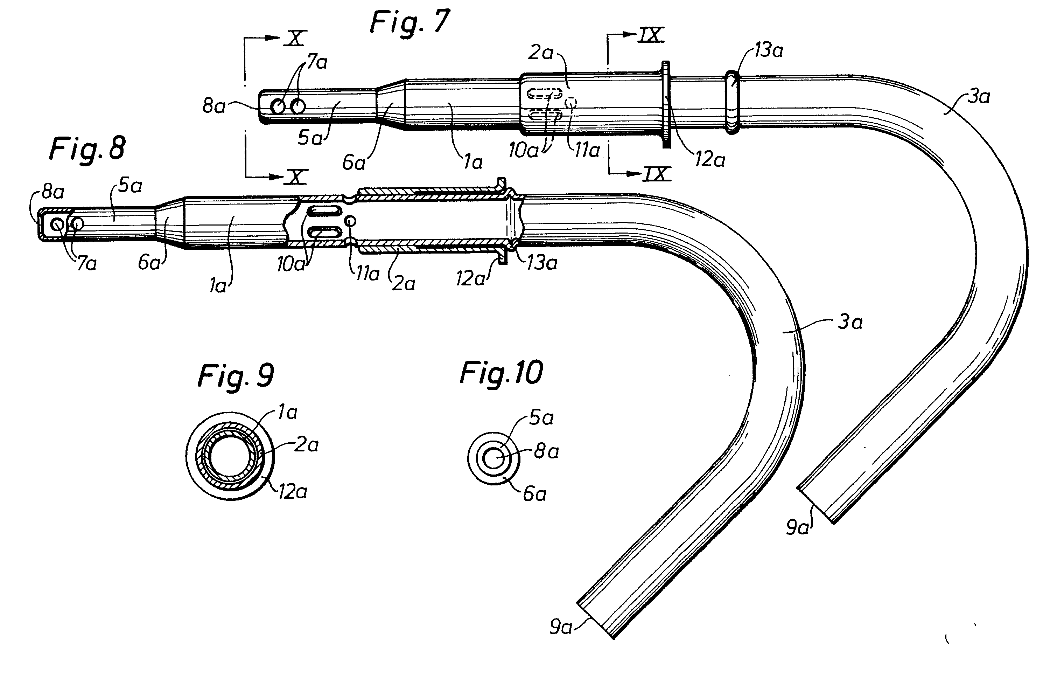

In fig 6-10 is shown a slightly simplified embodiment of the subject of the invention. Its design and function are substantially the same as for the catheter described above. For this reason the same reference numerals have been used for corresponding parts, but with the additional letter a. Thus 1a designates the main part of the catheter and 2a its slide. The bellows-like part 3, however, has been substituted by a plain part 3a which may be either rigid or bendable to the position shown. This catheter too, of course, can be provided with an evacuating arrangement, even though none is shown in the drawing. Like the catheter shown in fig 1-5 this catheter too can be used in essentially the same manner as the catheter described in the American patent 4 129 129, but without the disadvantages of the same.

Naturally the invention is not limited simply to the embodiments described above, but may be varied within the scope of the following claims.

Claims

1. A catheter comprising at least one inlet opening (7,8) at its one end, at least one outlet opening (9) at its other end and at least one intermediate opening (10,11) in between, which is adapted so that it can be closed whilst the said inlet and outlet openings are kept open, the said intermediate opening or intermediate openings (10,11) being arranged on an essentially cylindrical part (1) of the catheter, **char-**

acterized in that a slide (2) is arranged displaceable thereover for the opening and closing of this intermediate opening or these openings.

2. A catheter in accordance with claim 1 **characterized** in that the slide (2) is in the form of a cylindrical sleeve.

3. A catheter in accordance with claim 1, **characterized** in that the slide (2) is provided with a gripping part (12) in the form of a radially projecting part or flange.

4. A catheter in accordance with anyone of the preceding claims, **characterized** in that the actual catheter is provided with at least one stop element (13) determining one end position of the slide.

5. A catheter in accordance with anyone of the preceding claims, **characterized** in that its inlet end is designed as a circular-cylindrical part (5) of a smaller diameter.

6. A catheter in accordance with claim 4 or 5, **characterized** in that the said stop element is produced by means of upsetting of a heated part of the actual catheter to the shape of an all-around, radially outwards directed flange.

7. A catheter in accordance with anyone of the preceding claims, **characterized** in that its said other end is directed or directable at an angle in relation to its said first end.

8. A catheter in accordance with claim 7, **characterized** in that it is bendable with the help of a bellows-like intermediate part (3).

9. A catheter in accordance with anyone of the preceding claims, **characterized** in that it is provided with an evacuating arrangement (4) for the withdrawal of any air possibly accumulated between the blood and any priming fluid introduced.

10. A catheter in accordance with claims 8 and 9, **characterized** in that the evacuating arrangement (4) is constituted of a check valve through which the air can be pressed out using the bellows-like intermediate apart (3) as a pump.

11. A catheter in accordance with claim 9, **characterized** in that the evacuating arrangement (4) is constituted of an injection port or the like which is adapted so that it can be coupled to an external pump.

12. A catheter in accordance with claim 11, **characterized** in that the evacuating arrangement (4) comprises an elastic membrane, plug or the like, which is adapted so that it can be coupled to an external pump.

## Revendications

1. Cathéter comprenant au moins un orifice d'entrée (7,8) à une extrémité, au moins un orifice de sortie (9) à son autre extrémité et au moins un orifice intermédiaire (10,11) entre lesdites extrémités, qui est prévu de sorte qu'il peut être fermé pendant que les dits orifices d'entrée et de sortie sont maintenus ouverts, ledit orifice intermédiaire ou lesdits orifices intermédiaires (10,11) étant disposés sur une partie sensiblement cylindrique (1) du cathéter, caractérisé en ce qu'un coulisseau (2) est monté de façon déplaçable sur le cathéter pour l'ouverture et la fermeture de cet orifice intermédiaire ou de ces orifices intermédiaires.

2. Cathéter suivant la revendication 1, caractérisé en ce que le coulisseau (2) est sous la forme d'un manchon cylindrique.

3. Cathéter suivant la revendication 1, caractérisé en ce que le coulisseau (2) comporte une partie de préhension (12) sous la forme d'une partie ou collerette radialement en saillie.

4. Cathéter suivant l'une quelconque des revendications précédentes, caractérisé en ce que le cathéter effectif comporte au moins un élément de butée (13) déterminant une position extrême du coulisseau.

5. Cathéter suivant l'une quelconque des revendications précédentes, caractérisé en ce que son extrémité d'entrée est prévue comme une partie cylindrique circulaire (5) de plus petit diamètre.

6. Cathéter suivant la revendication 4 ou 5, caractérisé en ce que ledit élément de butée est produit par refoulement d'une partie chauffée du cathéter effectif à la forme d'une collerette périphérique dirigée radialement vers l'extérieur.

7. Cathéter suivant l'une quelconque des revendications précédentes, caractérisé en ce que sadite autre extrémité est dirigée ou peut être dirigée suivant un angle par rapport à sadite

premiére extrémité.

8. Cathéter suivant la revendication 7, caractérisé en ce qu'il peut être plié à l'aide d'une partie intermédiaire de type soufflet (3).

9. Cathéter suivant l'une quelconque des revendications précédentes, caractérisé en ce qu'il comporte un dispositif de purge (4) pour l'extraction de tout air éventuellement accumulé entre le sang et un fluide d'amorçage introduit.

10. Cathéter suivant les revendications 8 et 9, caractérisé en ce que le dispositif de purge (4) est constitué d'un clapet à travers lequel l'air peut être chassé par pression en utilisant la partie intermédiaire de type soufflet (3) comme une pompe.

11. Cathéter suivant la revendication 9, caractérisé en ce que le dispositif de purge (4) est constitué d'une tubulure d'injection ou analogue qui est conçue de sorte qu'elle peut être raccordée à une pompe extérieure.

12. Cathéter suivant la revendication 11, caractérisé en ce que le dispositif de purge (4) comprend une membrane élastique, un bouchon ou analogue, qui est conçu de sorte qu'il peut être raccordé à une pompe extérieure.

## Ansprüche

1. Katheter mit zumindest einer Einlaßöffnung (7, 8) an seinem einen Ende, zumindest einer Auslaßöffnung (9) an seinem anderen Ende und zumindest einer Zwischenöffnung (10, 11) dazwischen, welche so ausgelegt ist, daß sie verschlossen werden kann, während die Einlaß- und die Auslaßöffnungen offengehalten werden, wobei die Zwischenöffnung oder Zwischenöffnungen (10, 11) an einem im wesentlichen zylindrischen Teil (1) des Katheters angeordnet sind, dadurch gekennzeichnet, daß der Schieber (2) darüber verschiebbar für das Öffnen und Schließen dieser Zwischenöffnung oder dieser Öffnungen angeordnet ist.

2. Katheter nach Anspruch 1, dadurch gekennzeichnet, daß der Schieber (2) die Form einer zylindrischen Hülse hat.

3. Katheter nach Anspruch 1, dadurch gekennzeichnet, daß der Schieber (2) mit einem Griffteil (12) in Form eines radial vorspringenden Teiles oder Flansches versehen ist.

4. Katheter nach einem der vorstehenden Ansprüche, dadurch gekennzeichnet, daß der jeweilige Katheter mit zumindest einem Halteelement (13) versehen ist, welches eine Endstellung des Schiebers festlegt.

5. Katheter nach einem der vorstehenden Ansprüche, dadurch gekennzeichnet, daß sein Einlaßende als kreiszylindrischer Teil (5) mit kleinerem Durchmesser gestaltet ist.

6. Katheter nach Anspruch 4 oder 5, dadurch gekennzeichnet, daß das Halteelement durch Stauchen eines erhitzten Teils des jeweiligen Katheters zu der Form eines ganz umlaufenden, in radialer Richtung nach außen gerichteten Flansches erzeugt ist.

7. Katheter nach einem der vorstehenden Ansprüche, dadurch gekennzeichnet, daß sein anderes Ende bezüglich des ersten Endes unter einem Winkel ausgerichtet bzw. ausrichtbar ist.

8. Katheter nach Anspruch 7, dadurch gekennzeichnet, daß er mit Hilfe eines balgartigen Zwischenteiles (3) biegbar ist.

9. Katheter nach einem der vorstehenden Ansprüche, dadurch gekennzeichnet, daß er mit einer Auspumpanordnung (4) für das Abziehen jeglicher Luft versehen ist, welche sich möglicherweise zwischen dem Blut und irgendeinem eingeführten Ansaugfluid angesammelt hat.

10. Katheter nach den Ansprüchen 8 und 9, dadurch gekennzeichnet, daß die Auspumpanordnung (4) aus einem Rückschlagventil besteht, durch welches die Luft unter Verwendung des balgartigen Zwischenteiles (3) als Pumpe herausgedrückt werden kann.

11. Katheter nach Anspruch 9, dadurch gekennzeichnet, daß die Auspumpanordnung (4) aus einem Injektionsanschluß oder dergleichen besteht, welcher so ausgelegt ist, daß er an eine äußere Pumpe angeschlossen werden kann.

12. Katheter nach Anspruch 11, dadurch gekennzeichnet, daß die Auspumpanordnung (4) eine elastische Membran, einen Stopfen oder dergleichen aufweist, welcher so ausgelegt ist, daß er an eine äußere Pumpe angeschlossen werden kann.

Fig. 1

Fig. 2

Fig. 3

Fig. 4

Fig. 5

## Fig. 6

Fig. 7

Fig. 8

Fig. 9

Fig. 10

EP 0 230 040 B1